# EUROPEAN PATENT APPLICATION

(11) **EP 1 155 661 A1**
(43) Date of publication of application: **21.11.2001**
(21) Application number: 00902963.8
(22) Date of filing: 15.02.2000
(51) Int. Cl.: A61B 17/58

(54) **BONE FRACTURE THERAPEUTIC IMPLEMENT**

(30) Priority: 15.02.1999 JP 7825399; 05.03.1999 JP 10307099; 05.04.1999 JP 13314099
(71) Applicant: Ishibashi, Toru, Fukuoka-shi, Fukuoka 810-0041 (JP)
(72) Inventor: ISHIBASHI, Rika, Fukuoka-shi Fukuoka 810-0041 (JP)
(74) Representative: Harrison, David Christopher
(86) International application number: JP0000813
(87) International publication number: WO0047119

(57) **Abstract**

An intramedullary nail type of fracture therapeutic appliance especially suitable for treatment of fractures of weight-bearing long tubular bones, characterized by comprising: a nail member which is a cylindrical body having a length capable of extending from the proximal end of the target bone beyond the fracture point and which has a plurality of openings provided in a wall thereof so as to pass through the wall; at least one linear member having a tip end portion which has a diameter capable of passing through the openings from the lumen of the nail member to the outside and a length enough to pass through the cortex diagonally in the distal direction, and a trunk portion continuing from the tip end portion to extend up to the proximal end of the target bone, the number of the linear members being equal to or less than the number of the openings; and a guide member provided with at least one guide path which can house the linear member in the lumen thereof so as to receive it therethrough and which can guide the tip end portion of the linear member to the corresponding opening diagonally in the distal direction, and which makes contact with the inner surface of the nail member at at least two areas on the outer surface thereof.

## Description

### Technical Field

The present invention relates to an intramedullary nail type of fracture therapeutic appliance, and more particularly, an intramedullary nail type of fracture therapeutic appliance suitable for treatment of fractures of weight-bearing long tubular bones.

### Background Art

Fractures are the most frequently occurring type of injury among all types of injuries, and their healing requires the longest amount of time. Since weight-bearing long tubular bones such as the femur and tibia are organs that are required for people to walk, when these bones are fractured, the patient is unable to stand or walk until the bones have been repaired to an extent to which is the bones are again able to support body weight.

Although conjugative surgery of these weight-bearing long tubular bones is broadly divided into the use of a plate and the use of an intramedullary nail, since the intramedullary nail itself is able to support body weight in place of the bone, the patient is able to walk before healing of the bone occurs. Moreover, since it is not necessary to directly open the fractured site by surgery, there is no surgical damage to the fractured site. As a result, since this offers the advantage of rapid bone healing and so forth, the intramedullary nail has come to constitute the mainstream of fracture therapeutic appliances in conjugative surgery of long tubular bones.

However, the application range of early intramedullary nails was limited to comparatively simple fractures, while also being limited to treatment of fractures in the vicinity of the central portion of long tubular bones since the nail rotated at the fracture point and was unable to prevent shortening at the fracture point of unstable fractures.

A traverse immobilization type of intramedullary nail was developed to overcome these disadvantages. As a result, it became possible to apply intramedullary nails to unstable fractures of the diaphysis (in which the fracture point was shattered, fractured into three or four large fragments or fractured obliquely), complete transverse fractures of long tubular bones (which caused rotation in the case of conventional intramedullary nails) and fractures of long tubular bones in the vicinity of joints (they are currently used overwhelmingly as long tubular bone fracture appliances for the lower extremities).

However, this traverse immmovilization type of of intramedullary nails also had the problems indicated below.
1. Since a bone screw for traverse immobilization must be inserted from outside the bone through the intramedullary nail, it is necessary to cause invasion of the skin, fascia and tunica muscularis at least at the site of traverse immobilization. Consequently, time is required for bending rehabilitation of the knee joint caused by muscle damage following surgery or pain persists for a long time caused by adhesions at the same portion.
2. Drilling (using a radiolucent drill) while monitoring the locations of the traverse immobilization holes of the intramedullary nail by X-ray imaging and the use of a complex guide assembly are required, resulting in a complex operation technique that also takes considerable time. The prolonged duration of X-ray exposure places a considerable burden on not only the patient, but also the health care personnel as well.
3. It is necessary to again cause invasion of the site of traverse immmovilization when the nail is removed (and considerable surgical invasion must be applied that is naturally far greater than that during bone conjugation to deep locations of the tunica muscularis of the thigh region and so forth since the screws must be found and the relevant sites must be exposed), and is not uncommon that the bone screws also become damaged.

Although reports providing solutions to these new problems have been disclosed in, for example, Japanese Provisional Patent Publication No. Hei 8-103453, Japanese Provisional Patent Publication No. Hei 10-57398 and Japanese Provisional Patent Publication No. Hei 10-216150, a clinically satisfactory solution has not been obtained yet.

### DISCLOSURE OF THE INVENTION

The object of the present invention is to solve the problems of intramedullary nail types of fracture therapeutic appliances of the prior art, namely problems associated with surgical invasion, irradiation exposure and removal of the nail, along with providing an intramedullary nail type of fracture therapeutic appliance capable of rigidly immobilizing the fracture site using a simpler procedure.

Namely, the present invention relates to a fracture therapeutic appliance inserted into the bone marrow, which comprises a nail member in the form of a cylinder having a length that allows it to extend in the target bone from the proximal end thereof beyond the fracture point, and having a plurality of openings in its wall that pass through said wall; at least one linear member having a tip end portion, which has a diameter that enables it to pass through said openings from the lumen of said nail member to the outside and a length that allows it to pass through the cortex diagonally in the distal direction, and a trunk portion, which extends to the proximal end of said target bone continuing from said tip end portion, the number of said linear members being equal to or less than the number of said openings; and a guide member provided with at least one guide path, which together with being able to house said linear member in its lumen while allowing said linear member to be inserted through it, is capable of guiding the tip end portion of said linear member to said corresponding opening diagonally in the distal direction, and which makes contact with the inner surface of said nail member at at least two areas on its outer surface.

### BRIEF DESCRIPTION OF THE DRAWINGS

Fig. 1 is a perspective view showing the entire constitution of a nail member in a first embodiment of the present invention.
Fig. 2 is a cross-sectional view of the nail member shown in Fig. 1.
Fig. 3 is a perspective view showing the entire constitution of a guide member in a first embodiment of the present invention.
Fig. 4 is an enlarged cross-sectional view of a portion of the guide member shown in Fig. 3.
Fig. 5 is a longitudinal cross-sectional view (with a portion showing a side view) showing the state in which the fracture therapeutic appliance which is a first embodiment of the present invention is applied to the treatment of a fractured femur.
Fig. 6 is a perspective view showing the entire constitution of a nail member in a second embodiment of the present invention.
Fig. 7 is a perspective view showing the entire constitution of a guide member in a second embodiment of the present invention.
Fig. 8 is a perspective view showing the entire constitution of a guide member in a third embodiment of the present invention.
Fig. 9 is a horizontal cross-sectional view of the guide member shown in Fig. 8.
Fig. 10 is a longitudinal cross-sectional view (with a portion showing a side view) showing the state in which the fracture therapeutic appliance which is a third embodiment of the present invention is applied to the treatment of a fractured femur.
Fig. 11 is a longitudinal cross-sectional view of a cap member as an accessory that is applied to the nail member of the fracture therapeutic appliance of the present invention.
Fig. 12 is a perspective view showing the entire constitution of a guide member in a fourth embodiment of the present invention.
Fig. 13 is a horizontal cross-sectional view of the guide member shown in Fig. 12.
Fig. 14 is a side view showing the engaged state of a nail member and linear member in a fourth embodiment of the present invention.
Fig. 15 is a longitudinal cross-sectional view (with a portion showing a side view) showing the state in which the fracture therapeutic appliance which is a fourth embodiment of the present invention is applied to the treatment of a fractured humerus.

### BEST MODE FOR CARRYING OUT THE INVENTION

The following provides a detailed explanation of the present invention with reference to the drawings showing a portion of the embodiments of the present invention.

To begin with, the three basic members that compose the fracture therapeutic appliance of the present invention consist of a nail member (10, 110, 410) that functions as a structural member of the present therapeutic appliance that is inserted into the bone marrow; a linear member (30) that functions as a rotation preventing member of the fractured bone and a member which immobilizes said fractured bone to a nail member; and a guide member (20, 120, 220, 320) that functions as a member which guides the tip end of said linear member to a prescribed location of the bone where it is to be driven in.

The basic characteristics of these three members are as described below.

### 1. Nail Member

This member is a cylindrical body having a length that enables it to extend passing from the proximal end through the fractured site of the bone in which it is inserted (hereinafter referred to as the "target bone"), and which has a plurality of openings (11, 111) in its wall that pass through said wall. In the attached drawings, although said openings are depicted as being round, the present invention does not restrict their shape, and may be in shape of, for example, slots or slits provided they are of a shape that allows the linear member (30) to be described later to pass through diagonally in the distal direction. Furthermore, a large number of said openings are preferably provided so as to be able to cope with numerous cases. However, their number should be determined while giving considerations so as not to decrease the strength of the nail member itself. Materials generally used as medical materials for orthopedic surgery are used for the material of said nail member, examples of materials which should be used including titanium, stainless steel, Vitallium, alumina and zirconia.

### 2. Linear Member

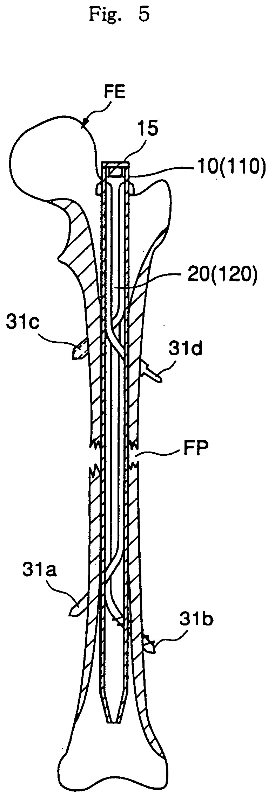

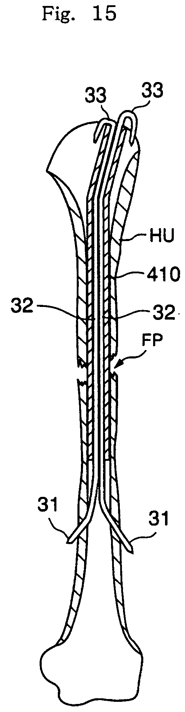

This member has a tip end portion (31, 531), which has a diameter that is able to pass through the openings of said nail member from the lumen to the outside and a length that is able to pass through cortex diagonally in the distal direction and engage with the inner edges of the openings of said nail member, and a trunk portion (32), which extends at least to the proximal end of the target bone continuing from said tip end portion (this member is depicted with a side view in Fig. 5, Fig. 10 and Fig. 15). Based on the function required of this member, and particularly the function of preventing rotation of bone fragments after treatment, the number of this member should be at least one in the case of a simple fracture of a weight-bearing long tubular bone (although one each is required for bone fragments on both sides of the fracture point (FP), in the case at least one rotation preventing appliance (not shown), such as a fin-shaped member extending for a prescribed length in the axial direction of said nail member, being provided at a site on the outer surface of the proximal side of the nail member (10, 110, 410) and in opposition to the cortex inner surface of the bone fragment on the proximal side, since this cuts into said bone fragment on the proximal side and said nail member stops said bone fragment on the proximal side from rotating, its objective is achieved with only one such member for engaging with the bone fragment on the distal side). However, the number of this member should naturally not exceed the number of openings provided in said nail member.

A typical example of the material of this member is a Kirschner's wire known in the field of orthopedic surgery (while other examples include titanium and Vitallium).
Said linear member is basically a single linear member in which its tip end portion (31) and its trunk portion (32) are connected. Since said linear member is used by guiding through the lumen of a guide path of a guide member to be described later from its proximal end towards its distal end, and in the state in which it is deformed so that the portion that migrates from said tip end portion to said trunk portion depicts a gradual arc, being forcibly passed through cortex C from the inside to the outside by, for example, rotating its proximal end to cause the tip of said tip end portion to pass through said cortex C, preferably has suitable elasticity and toughness from the viewpoint of transferring the rotational force of said proximal end to said tip end portion, while also having suitable shear resistance in order to fulfill the function of immobilizing a fractured bone fragment to said nail member.

Furthermore, in order to facilitate the cutting of this member into cortex C and its penetration, the tip is preferably sharp (see reference numerals 31a to 31d of Fig. 5). In addition, in order to further facilitate this cutting and penetration while also imparting force that resists extraction, it is more preferable to provide external threads (and preferably tapping screw threads) into said tip end portion (see reference numeral 31b of Fig. 5). (When employed in this state, the appliance is also effective in cases of fractures in which there is a third or additional number of bone fragments. Namely, after screwing in said tip end portion into a bone fragment that has been restored to its inherent position manually or by other means, by pulling on the proximal end of said linear member, said bone fragment and other major bone fragments can be aligned and immobilized.)

Although the explanation thus far has focused on that in which linear member (30) is composed of a single member that is uniform from its proximal end to its distal end, the functions required of its tip end portion (31) and trunk portion (32) are different. Namely, said tip end portion is required to be able to pass through and be removed from cortex C while rotating as well as be able to oppose shearing load in collaboration with the inner surfaces of the openings of said nail member, said trunk member is required to be able to transmit rotational force applied to the proximal end to said tip end portion, and the portion that migrates from said tip end portion to said trunk portion is required to be able to transmit said rotational force smoothly to said tip end portion in a state in which it is deformed into the shape of an arc by said guide member. Thus, in order to be able to fulfill these required functions, it is also permitted that these portions be in the form of individual members (such as those composed of materials having a balance between rigidity and toughness for said tip end portion, applying the threads themselves, or using a material having ample elasticity for said migrating portion) or that the properties of these portions be changed (for example, by changing the diameter of said tip end portion, namely by making the portion connected to said migrating portion of a large diameter with the emphasis on resistance to shearing load and making the portion that forms its end of a small diameter with the emphasis on ease of penetration and extraction from the cortex (see reference numeral 31d of Fig. 5), by performing heat treatment on said tip end portion, or by enhancing hardness by fitting a hollow member of high hardness around the outer surface of said tip end portion (see reference numeral 31c of Fig. 5).

### 3. Guide Member

This member is provided with at least one guide path (21, 121, 221, 321) that is able to house said linear member so as to be able to pass through its lumen while also guiding the tip end portion of said linear member to the corresponding opening of said nail member diagonally in the distal direction. (Said guide path is provided with at least one curved portion in order to perform this guidance. The portion that migrates from the tip end portion to the trunk portion of the said linear member at the time of use corresponds to this curved portion. Thus, the diameter of the guide path and curvature of the curved portion should be set to values that allow passage of the tip end portion of said linear member.) In addition, at least two areas on its outer surface are in contact with the inner surface of said nail member (when the inner surface of said nail member is substantially circular and said guide member is composed of a plate-shaped member, the two side faces correspond to these areas, and in the case of said guide member having a polygonal horizontal cross-section (which is circular in the extreme form), the side or side face that connects its upper and lower apices correspond to these areas). Furthermore, preferable examples of the material of this member include plastic represented by high-density polyethylene, stainless steel, titanium, alumina, zirconia and hydroxyapatite.

The following provides an explanation of a typical embodiment of the present invention.

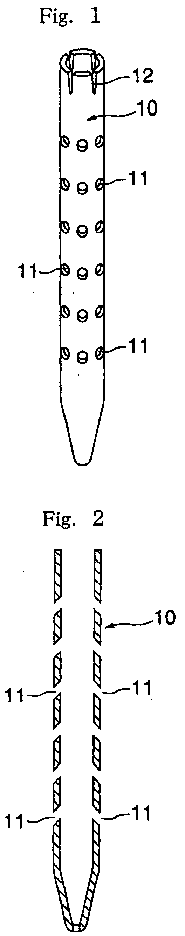

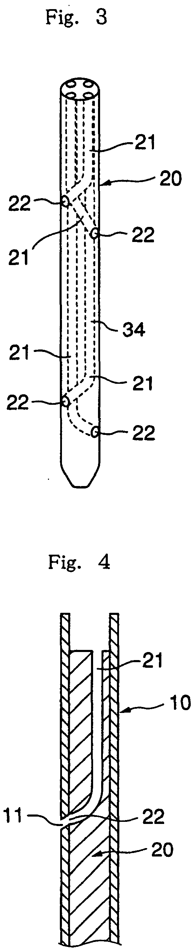

To begin with, a first embodiment (see Figs. 1 to 5 and Fig. 11; furthermore, the depiction of guide member (20) has been omitted from Fig. 5 for the sake of simplicity) is fracture therapeutic appliance of the type in which guide member (20) is left in the body until the fracture has completely healed (or more accurately, until the nail is removed).

Here, nail member (10) is a cylindrical body having an outer diameter that corresponds to the minimum inner diameter of the bone marrow in which it is inserted (portion in which the cancellous bone is present), the horizontal cross-section of which is substantially circular (the number of openings 11 formed is larger than the number of required linear members (30) depending on the case to which it is to be applied so as to be able to be shared among the greatest common measure of cases). Furthermore, although the tip end of nail member (10) is of a form that gradually narrows to a tapered shape in the drawings, this is for enabling it to smoothly pass through the narrow portion formed by the fracture (portion to be joined at which the axis has shifted between the bone fragment on the distal side and the bone fragment on the proximal side).

On the other hand, guide member (20) is a cylindrical body having an outer diameter such that its outer surface substantially makes contact with the inner surface of said nail member, its horizontal cross-section being substantially circular. (A plurality of guide paths (21) are formed within guide member (20). Although a mode is shown in the drawings in which said guide paths are bored and formed in a cylindrical guide member, the mode of said guide paths is not limited to this. For example, a prescribed number of tubular bodies equivalent to the number of said guide paths may be fixed within a cylindrical body, or they may be fixed to a supporting member comprised of disks on which these tubular bodies are arranged on the upper and lower ends (having an outer diameter such that their peripheral surfaces make contact with the inner surface of said nail member) and a rod member that connects both of these disks.) What is important here is that tip end portion (22) of said guide path is inclined diagonally in the distal direction so that tip end portion (31) of linear member (30) to be described later is guided diagonally in the distal direction relative to the cortex through which it is to pass, that tip end portion (22) of said guide path be formed so its axis aligns with the opening of said nail member to which the tip of said tip end portion corresponds, and that tip end portion (22) of said guide path have a diameter and curvature that enables the curved portion to smoothly guide said linear member. Furthermore, the number of said guide paths is equal to or greater than the number of said linear members.

Incidentally, when considering the case of applying to a fractured femur of an adult male (simple fracture), the main specifications of the fracture therapeutic appliance are as shown below.
1) Nail Member
   · Material : Stainless steel (or the previously exemplified materials may be used in its place)
   · Outer diameter : 100 - 160 mm
   · length : 150 - 450 mm
   · thickness : 1 - 3 mm
   · Number of openings : 1 - 12
2) Guide Member
   · Material : Stainless steel (or the previously exemplified materials may be used in its place)
   · Outer diameter : 80 - 150 mm
   · length : 150 - 450 mm
   · Number of guide paths : 1 - 8
3) Linear Member
   · Material : Kirschner's wire (or the previously exemplified materials may be used in its place)
   · Number of linear members : 1 - 8

During use, said guide member is fit into the lumen of said nail member so that the tip end of the prescribed guide path of said guide member corresponds to the prescribed opening of said nail member.

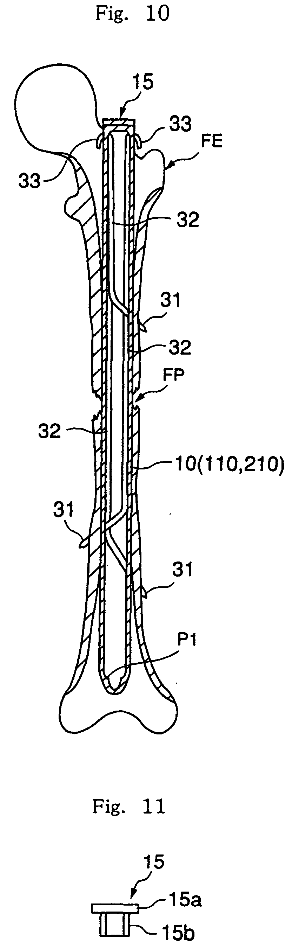

Furthermore, this type of therapeutic appliance is used in the manner described below (an explanation is provided using as an example the case of a simple fracture near the central portion of the femur).
1) After making an incision in the hip joint region to expose the fossa trochanteria, nail member (10), in which a prescribed guide member (20) is fit into its lumen at a prescribed angle and to a prescribed position, is inserted into the bone marrow of the femur (FE) from the incision at said fossa trochanteria. As a result, said nail member is positioned extending from the proximal side to the distal side of the fracture point (FP).
2) Linear member (30) having a prescribed length (the mode of tip end portion (31) thereof should be suitably selected from the above-mentioned modes including each of the modes (31a) to (31d) shown in Fig. 5, and this is to apply similarly hereinafter) is then inserted into a prescribed guide path (21) from the proximal end of said guide member. As a result, tip end portion (31) of said linear member passes through the curved portion of said guide member, protrudes through opening (11) of said nail member, and its tip strikes the inner surface of cortex C in a state in which it is inclined diagonally in the distal direction.
3) The proximal end of said linear member is connected to a rotator (examples of which include the "Commando" made by Striker, and the "Mini Driver" or "Maxi Driver" made by 3M, to apply similarly hereinafter) to rotate said linear member. As a result, tip end portion (31) of said linear member enters the cortex diagonally in the distal direction.
4) When the tip of said linear member protrudes through the outer surface of the cortex, rotation of said rotator is discontinued. In this manner, the application of said linear member so as to pass through the cortex is a major characteristic of the present invention.
5) The proximal end of said linear member is caught on a notch (12) formed in the proximal end of said nail member (see Figs. 1 and 5), and the proximal end of said linear member located above said notch is bent outward.
6) After performing the same treatment for the prescribed number of linear members, the proximal end of said nail member is covered with a cap member (15) (see Figs. 5 and 11). (Here, it is preferable that threads be provided in the outer surface of cylinder portion 15b of said cap member and in the inner surface of the proximal end of said nail member, respectively. This is done to more reliably prevent the proximal end of said linear member from unexpectedly coming out of said notch.)

Furthermore, when healing is made, an incision is again made in the hip joint region after which linear member (30) and nail member (10) (with the guide member still inserted) are extracted in that order (said linear member is extracted by applying rotation in the direction opposite that during application).

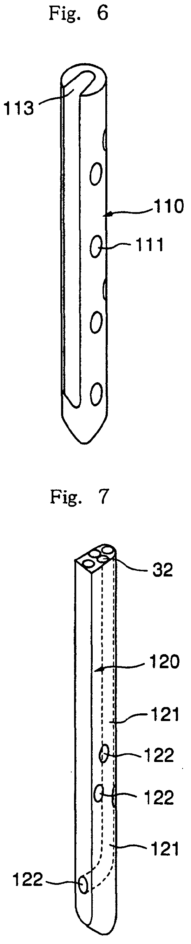

Next, a second embodiment (see Figs. 6 and 7) is a therapeutic appliance of the same type as that of the first embodiment, and its constitution and method of use are substantially the same as those of the first embodiment with the exception of nail member (110) having a notch (113) over its entire length in its lengthwise direction, and said notch having an angle of 60 degrees or less for the angle around the axis of said nail member (for securing a surface that forms openings (111) of said nail member and preventing guide member (120) inserted into the lumen of said nail member from falling out). Furthermore, with respect to other reference numerals shown in the drawings, (121) designates a guide path of said guide member, and (122) designates the tip end portion of said guide path (although notch (12) provided in the proximal end of said nail member as explained in the first embodiment is not clearly indicated, it may be provided in the same manner as the first embodiment, and this applies similarly hereinafter).

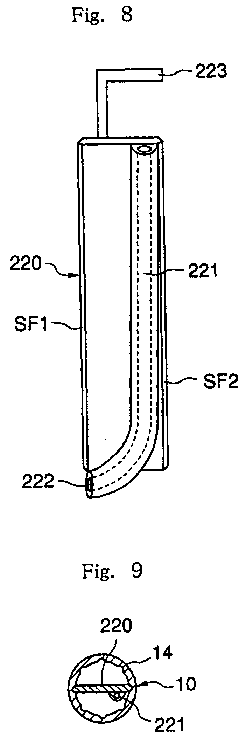

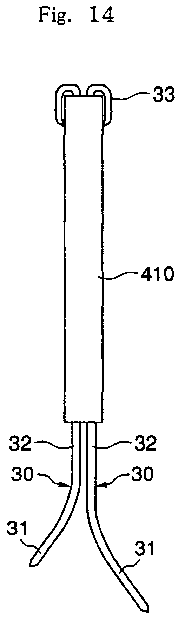

The fracture therapeutic appliances described below differ from the first and second embodiments explained thus far in that they are of the type in which the guide member is extracted at completion of insertion of the fracture therapeutic appliance in the body (see Figs. 8 to 15).

Their basic characteristics consist of the guide member (220, 320) being a substantially plate-shaped member, having a single guide path (221, 321) on either the front surface or rear surface of said plate-shaped member, or within said plate-shaped member (for example, providing a tubular path along the surface of a plate-like body, or either combining in the form of a plate-tube-plate assembly a tubular path or forming a tubular path by boring within the plate-like body), and making contact with the inner surface of the nail member (10, 110) on two side faces of said plate-shaped member (the nail member (10, 110) and linear member (30) explained in the first and second embodiments should be used for said nail member and linear member for these type of the fracture therapeutic appliances as a general rule).

A specific embodiment (third embodiment) is as shown in Figs. 8 and 9. Furthermore, reference numeral (222) designates the tip end portion of said guide path, while reference numeral (223) designates a handle for extracting said guide member.

In addition, reference numeral (14) designates a member that serves as a guide for fitting said guide member into said nail member, and more specifically, designates a groove extending linearly in the lengthwise direction of said nail member in its inner surface. On the other hand, a projection (not shown) that contacts and engages with this groove is formed in two side faces SF1 and SF2 of said guide member (this projection and groove may also have the opposite relationship, namely said projection may be provided on said nail member and said groove may be provided in said guide member). If such a groove or projection is formed on the inner surface of said nail member such that the tip end portion (222) of a guide path (221), provided in a guide member that contacts and engages with that groove or projection, corresponds to a prescribed opening (11) of said nail member, tip end portion (31) of a prescribed linear member can be guided to the prescribed opening (11) of said nail member. In addition, positioning of said guide member in the lengthwise direction of said nail member can be performed by, for example, attaching a scale to the side face of said guide member.

Treatment of a fracture using this type of guide member should be performed in the same manner as the first embodiment with the exception of not inserting said guide member into the lumen of said nail member prior to inserting said nail member into the bone marrow, removing or inserting said guide member from said nail member (or into said nail member) for a number of times corresponding to the number of said linear members, and extracting said guide member from said nail member when insertion of said therapeutic appliance into the body is completed. Furthermore, the mode shown in Fig. 10 shows this type of the fracture therapeutic appliance at completion of its insertion in the body. In this mode, guide member (220) is not shown in the drawing since it is not remaining in the lumen of the nail member (10, 110, 210).

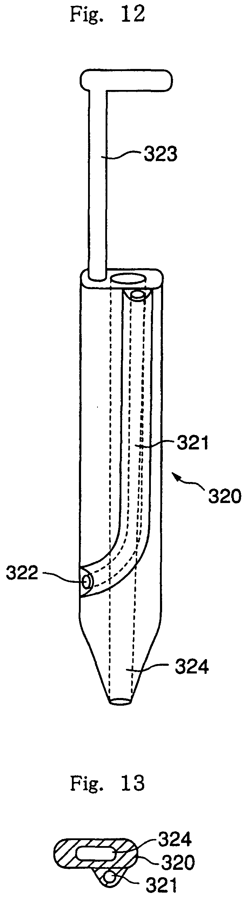

The mode shown in Figs. 12 to 15 shows another specific mode of a fracture therapeutic appliance of this type (fourth embodiment).

The therapeutic appliance of this mode is used to treat fractures at a site to which a load is not applied or at which the application of a load does not cause concern.

The major constituents of this embodiment that differ from those of the previous embodiments are that the nail member (410) is in the form of a cylindrical body that does not have openings (11), it has at least one linear member (30, 530) (rotation of the bone fragment can be inhibited with only one linear member if a rotation preventing appliance is provided around the proximal side of the nail member as was previously explained in the section entitled, "Nail Member"), the tip end portion (31, 531) of said linear member protrudes beyond the distal end of said nail member, said guide member is a plate-shaped member having one guide path (321) either disposed on its front or rear surface or provided within said guide member, and has a centering rod member path (324) extending linearly in its lengthwise direction inside (this path may also be in the form of a plate-tube-plate combination in the same manner as the third embodiment). (Furthermore, reference numeral (322) designates the tip end portion of said guide path, and reference numeral (323) designates a handle for extracting said guide member.)

In this mode, since contrivances have been made in said guide member itself for removing and inserting into the bone marrow and for positioning the tip end of said guide path, respectively (and more specifically, since path (324) and rod member inserted into said path (not shown) are respectively provided), it is not necessary to make the side face of said guide member contact and engage with the inner surface of said nail member as in the previously described third embodiment. Furthermore, positioning of the tip end of said guide path only requires that scales be attached in the lengthwise direction around said path and on the outside of the rod member, respectively. Naturally, a groove and projection may be respectively provided in the lengthwise direction in the surface of said path and on the surface of said rod member as in the third embodiment.

Incidentally, when considering the case of applying to a fractured humerus of an adult male (simple fracture), the main specifications of the fracture therapeutic appliance are as shown below.
1) Nail Member
   · Material : Stainless steel (or the previously exemplified materials may be used in its place)
   · Outer diameter : 100 - 160 mm
   · length : 100 - 400 mm
   · thickness : 1 - 3 mm
2) Guide Member
   · Material : Stainless steel (or the previously exemplified materials may be used in its place)
   · width : 60 - 140 mm
   · length : 100 - 350 mm
   · thickness : 1 - 5 mm
   · Material of guide paths :Stainless steel (or the previously exemplified materials may be used in its place)
3) Linear Member
   · Material : Kirschner's wire (or the previously exemplified materials may be used in its place)
   · Number of linear members : 1 - 12

The therapeutic appliance of this mode is used in the manner described below (explained using the example of a simple fracture of the central portion of the humerus).
1) After making an incision in the shoulder joint region to expose the greater tubercle of the humerus HU, a rod member (not shown) is inserted into the lumen of the humerus from the incision at said greater tubercle of the humerus, and then the guide member (320) is fitted onto said rod member through the path (324) at the prescribed angle and to the prescribed position (at least to a position beyond the fracture point FP).
2) Linear member (30) of a prescribed length is the inserted from the proximal end of said guide member. As a result, the tip of tip end portion (31) of said linear member (various modes can be selected for its mode, including the previously mentioned 31a to 31d) strikes the inner surface at a prescribed position of the cortex diagonally in the distal direction.
3) As a result of inserting into the lumen of said guide path, the proximal end of said linear member, in which the portion migrating from tip end portion (31) to trunk portion (32) is curved, is connected to the previously mentioned rotator to rotate said linear member. As a result, tip end portion (31) of said linear member enters the cortex diagonally in the distal direction.
4) Rotation of said rotator is discontinued when the tip of said linear member protrudes through the outer surface of the cortex.
5) Said guide member is extracted from said linear member.
6) Said guide member is again fitted onto said rod member while avoiding said linear member that has been previously applied.
7) The procedure from step 2) to step 6) is repeated for the prescribed number of linear members.
8) Following completion of the procedure of step 5) for the last linear member, nail member (410) is inserted into the lumen of the humerus so that all linear members are positioned within its lumen. As a result, said nail member is positioned from the proximal side to the distal side of the fracture point FP.
9) Finally, the proximal end of the said linear member is bent to the outside beyond the proximal end of said nail member. As a result, said linear member is anchored at the cortex on the distal side and the bone fragment on the proximal side by means of the nail member. Furthermore, as in the first to third embodiments, a mode may be employed in which notches (12) are provided in the proximal end of said nail member, and together with catching the bent proximal end of said linear member in these notches, cap member (15) is placed over (or screwed onto) the proximal end of said nail member. In addition, a different notch (not shown) equivalent to notch (12) may be additionally provided in the distal end of said nail member, and the tip end portion of said linear member may be made to pass through this notch.

Furthermore, when healing is made, an incision is again made in the shoulder joint region and the nail member and linear members are extracted in that order.

### INDUSTRIAL APPLICABILITY

According to the fracture therapeutic appliance of the present invention as explained above, since surgery is performed twice at the time of insertion of the therapeutic appliance and at the time of its extraction, and over an extremely limited region, treatment can be performed with an extremely low degree of invasiveness. Moreover, since the use of X-ray irradiation is only performed over a limited region and for a limited duration as a general rule, X-ray exposure does not present any particular problem. In addition, since the linear member of the present invention is immobilized in the cortex after diagonally passing through said cortex from the bone marrow side by means of openings in the nail member, immobilization of the bone fragments is extremely rigid. Moreover, since the accompanying procedure can be performed extremely easily with the guide member, the burden on the surgeon is significantly improved.

## Claims

1. A fracture therapeutic appliance inserted into the bone marrow, which comprises a nail member in the form of a cylinder having a length that allows it to extend in the target bone from the proximal end thereof beyond the fracture point, and having a plurality of openings in its wall that pass through said wall; at least one linear member having a tip end portion, which has a diameter that enables it to pass through said openings from the lumen of said nail member to the outside and a length that allows it to pass through the cortex diagonally in the distal direction, and a trunk portion, which extends to the proximal end of said target bone continuing from said tip end portion, the number of said linear members being equal to or less than the number of said openings; and a guide member provided with at least one guide path, which together with being able to house said linear member in its lumen while allowing said linear member to be inserted through it, is capable of guiding the tip end portion of said linear member to said corresponding opening diagonally in the distal direction, and which makes contact with the inner surface of said nail member at at least two areas on its outer surface.

2. The fracture therapeutic appliance according to claim 1, wherein said guide member has at least the same number of guide paths as the number of said linear members, and its outer surface contacts the inner surface of said nail member.

3. The fracture therapeutic appliance according to either claim 1 or 2, wherein said nail member has a notch extending along its entire length in the lengthwise direction, and said notch has an angle around the axis of said nail member of 60 degrees or less.

4. The fracture therapeutic appliance according to claim 1, wherein said guide member is a plate-shaped member, has one guide path provided on either its front or rear surface, or inside thereof, and contacts the inner surface of said nail member at two of its side faces.

5. The fracture therapeutic appliance according to claim 4, wherein together with providing a projection or groove on the inner surface of said nail member extending linearly in its lengthwise direction, a groove or projection is provided on the side surface of said guide member that contacts and engages with the projection or groove of said nail member and that extends linearly in its lengthwise direction.

6. The fracture therapeutic appliance according to any of claims 1 to 5, wherein said linear member is a linear Kirschner's wire.

7. The fracture therapeutic appliance according to claim 6, wherein said linear member has external threads provided in its tip end.

8. The fracture therapeutic appliance according to any of claims 1 to 5, wherein said nail member has a number of notches at least equal to the number of said linear members in the wall on the proximal end of said nail member.

9. The fracture therapeutic appliance according to claim 8, wherein said notches are able to anchor the proximal end of said bent linear member.

10. The fracture therapeutic appliance according to either claim 8 or 9, wherein said linear member is a linear Kirschner's wire.

11. The fracture therapeutic appliance according to claim 10, wherein said linear member has external threads provided in its tip end.

12. The fracture therapeutic appliance according to claim 1, wherein said nail member is a cylindrical body not having said openings and said appliance has at least one said linear member of which the tip end portion protrudes beyond the distal end of said nail member, and said guide member is a plate-shaped member having one guide path provided along either its front or rear surface, or inside thereof, and having a centering path that extends linearly in its lengthwise direction.
